**Europäisches Patentamt**

**European Patent Office**

**Office européen des brevets**

(19)

(11) Veröffentlichungsnummer: **0 184 695**
**B1**

(12) # EUROPÄISCHE PATENTSCHRIFT

(45) Veröffentlichungstag der Patentschrift:
08.02.89

(51) Int. Cl.⁴: **A61B 6/06**, G21K 1/04

(21) Anmeldenummer: 85114759.5

(22) Anmeldetag: **21.11.85**

(54) Primärstrahlenblende für Röntgenuntersuchungsgeräte.

(30) Priorität: **03.12.84 DE 3444058**

(43) Veröffentlichungstag der Anmeldung:
**18.06.86 Patentblatt 86/25**

(45) Bekanntmachung des Hinweises auf die Patenterteilung:
**08.02.89 Patentblatt 89/6**

(84) Benannte Vertragsstaaten:
**DE FR**

(56) Entgegenhaltungen:
**US-A- 3 980 407**

(73) Patentinhaber: **Siemens Aktiengesellschaft Berlin und München, Wittelsbacherplatz 2, D-8000 München 2(DE)**

(72) Erfinder: **Telorack, Gerhard, Röthenäckerstrasse 10, D-8521 Aurachtal(DE)**

## Beschreibung

Die Erfindung betrifft eine Primärstrahlenblende für Röntgenuntersuchungsgeräte mit mindestens einem Paar gegenläufig zueinander verstellbarer Blendenplatten.

Eine Primärstrahlenblende dieser Art ist in der DE-OS 1 800 879 beschrieben. Diese bekannte Primärstrahlenblende weist zwei Blendenplatten auf, die an ihren das Röntgenstrahlenbündel begrenzenden Kanten keilförmig verlaufen, so daß die Röntgenstrahlung im kantennahen Blendenbereich nicht vollständig absorbiert wird. Auf diese Weise ist einerseits eine gute Einblendung des diagnostisch relevanten Bildbereiches möglich, so daß kaum zu hell strahlende, keine Bildinformationen enthaltende Bildbereiche vorhanden sind und andererseits gewährleistet, daß beim Einführen von Instrumenten in das Untersuchungsobjekt kurz vor dem Eintreten in den diagnostisch relevanten Bereich eine Beobachtung auf einem Bildwiedergabegerät, insbesondere einem Monitor, möglich ist.

Bei der bekannten Primärstrahlenblende verlaufen die das Röntgenstrahlenbündel begrenzenden Blendenkanten geradlinig. In der Praxis entspricht dieser geradlinige Verlauf nicht immer der gewünschten Bildfeldbegrenzung. Insbesondere für die Einblendung des Herzens wäre ein gekrümmter Kantenverlauf wünschenswert, so daß das Bildfeld der Herzform optimal angepaßt werden kann.

Durch die US-PS 3 980 407 ist es bereits bekannt, ein Strahlenbündel wahlweise durch unterschiedlich geformte Kanten von übereinander hinwegbewegbaren Blendenplatten einzublenden. Auf diese Weise können mit denselben Blendenplatten unterschiedliche Formen des eingeblendeten Feldes erreicht werden. Es ist dabei jedoch nur möglich, entweder mit den beiden geknickten oder den beiden geradlinigen Kanten der beiden Blendenplatten einzublenden.

Der Erfindung liegt die Aufgabe zugrunde, eine Primärstrahlenblende der eingangs genannten Art so auszubilden, daß mit Hilfe von zwei Blendenplatten eine Vielzahl unterschiedlicher Bildfeldformen möglich ist, so daß beispielsweise wahlweise eine rechteckige und eine der Herzkontur entsprechende Einblendung möglich ist, die in bezug auf das jeweilige Untersuchungsobjekt individuell angepaßt werden kann.

Diese Aufgabe ist durch die im kennzeichnenden Teil von Anspruch 1 aufgeführten Merkmale gelöst.

Die Erfindung ist nachfolgend anhand eines in der Zeichnung dargestellten Ausführungsbeispieles näher erläutert. Es zeigen:

Fig. 1 die wesentlichen Teile einer Primärstrahlenblende zur Erläuterung des Erfindungsgedankens, und

Fig. 2 und 3 eine Einzelheit der Primärstrahlenblende gemäß Figur 1 in zwei verschiedenen Ansichten.

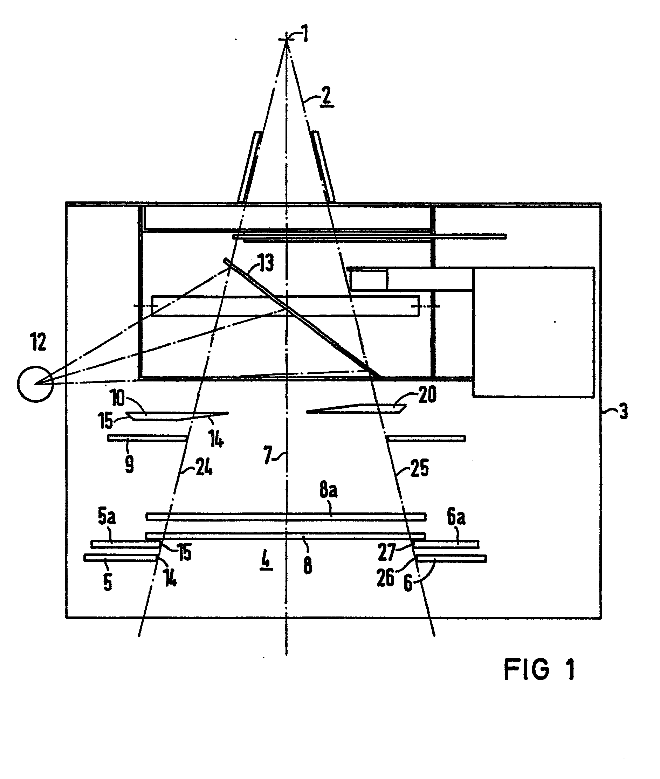

In der Figur 1 ist der Fokus 1 einer Röntgenröhre dargestellt, dessen Röntgenstrahlenbündel 2 durch eine Primärstrahlenblende 3 pyramidenförmig eingeblendet wird. Hierzu ist eine Rechteckblende 4 vorhanden, die zwei Blendenplattenpaare aufweist, deren Blendenplatten 5, 6, 5a, 6a gegenläufig zueinander verstellbar sind, so daß die Breite des Röntgenstrahlenbündels 2 einblendbar ist. Die oberen Blendenplatten 5a, 6a der Blendenplattenpaare werden dabei zur Ausrichtung auf den Fokus 1 jeweils etwas weiter zum Zentralstrahl 7 des Röntgenstrahlenbündels 2 bewegt als die unteren Blendenplatten 5, 6. Analog dazu sind zwei weitere Blendenplattenpaare vorhanden, die senkrecht zu den ersten Blendenplattenpaaren liegen und von denen in der Figur 1 die Blendenplatten 8, 8a sichtbar sind. Diese weiteren Blendenplattenpaare dienen zur Festlegung der Abmessung des Röntgenstrahlenbündels 2 in der anderen Dimension.

In Strahlenrichtung gesehen vor der Rechteckblende 4 ist eine Irisblende 9 vorgesehen, die die Form des Röntgenstrahlenbündels 2 durch Einschieben von Keilen über die Ecken der Rechteckblende 4 an die Form des Eingangsleuchtschirmes eines Röntgenbildverstärkers anpaßt.

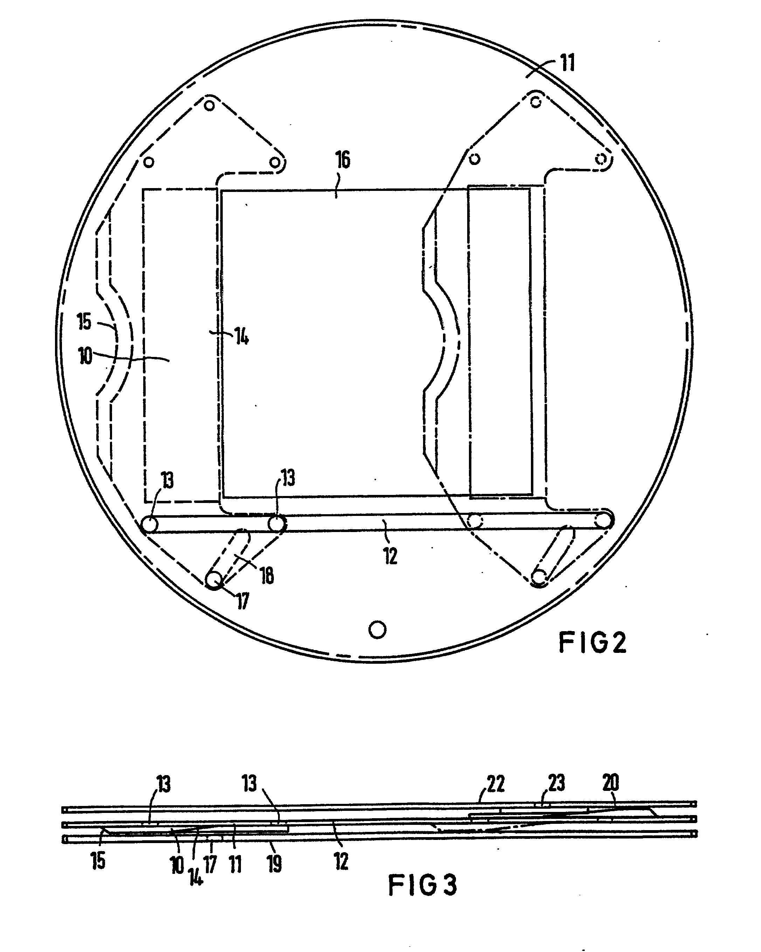

In Strahlenrichtung vor der Irisblende 9 sind zwei Blendenplatten 10, 20 vorhanden, die ebenfalls gegenläufig zum Zentralstrahl 7 verstellbar sind, und zwar in zwei im Abstand voneinanderliegenden Ebenen, so daß sie aneinander vorbeibewegt werden können. Der Stellweg für die Blendenplatten 10, 20 ist dabei so groß, daß durch jede Blendenplatte 10, 20 das Röntgenstrahlenbündel 2 wahlweise mit einer von zwei quer zum Stellweg liegenden Kanten begrenzbar ist. Dies ist nachfolgend anhand der Figuren 2 und 3 noch näher erläutert. Schließlich weist die Primärstrahlenblende 3 noch ein Lichtvisier mit einer Lichtquelle 12 und einem für Röntgenstrahlung durchlässigen Spiegel 13 auf, durch den das jeweils eingeblendete Feld auf dem Untersuchungsobjekt sichtbar gemacht werden kann.

Aus den Figuren 2 und 3 geht die Begrenzung des Röntgenstrahlenbündels 2 mit Hilfe der Blendenplatten 10 und 20 deutlicher hervor. In der Figur 2 ist dabei nur die linke Blendenplatte 10 dargestellt. Die Blendenplatte 10 ist auf einer ringförmigen Scheibe 11 längs eines Schlitzes 12 in der Scheibe 11 verschiebbar gelagert und in dem Schlitz 12 durch zwei Zapfen 13 geführt. Der Schlitz 12 ist so lang, daß das Röntgenstrahlenbündel 2 wahlweise mit der rechten Kante 14 oder der linken Kante 15 der Blendenplatte 10 begrenzt werden kann. Die maximale Blendenöffnung ist dabei durch eine rechteckige Öffnung 16 in der Scheibe 11 festgelegt. In den Figuren 2 und 3 ist die linke Endstellung der Blendenplatte 10 gestrichelt gezeichnet. Aus diesen Figuren geht hervor, daß die Blendenplatte 10 aus dieser Endstellung in eine strichpunktiert gezeichnete Stellung bewegt werden kann, in der nicht die Kante 14, sondern die Kante 15 das Röntgenstrahlenbündel 2 begrenzt. Die Kanten 14 und 15 haben dabei unterschiedliche Formen, so daß das jeweils eingeblendete Feld ebenfalls je nach Stellung der Blendenplatte 10 eine unterschiedliche Form aufweist. Die Kante 15 dient dabei zur Einblendung einer der Herzkontur entsprechenden Form und ist demgemäß gekrümmt, während die Kante 15 geradlinig verläuft. Die Blendenplatte 10 ist in bekannter Weise in ihren kantennahen Bereichen abgeschrägt und damit teiltransparent. Die Blendenplatte 11 ist spiegelbildlich dazu ausgebildet.

Die Verstellung der Blendenplatte 10 erfolgt durch einen an ihr befestigten Stift 17, der in einem Langloch 18 einer unter der Scheibe 11 liegenden Scheibe 19 geführt ist. Auch die Scheibe 19 weist wie eine über der Scheibe 11 liegende Scheibe 22 eine zentrische Öffnung zur Festlegung der maximalen Feldgröße auf. Die Scheibe 22 ist in Figur 2 der Übersichtlichkeit halber nicht gezeichnet. Die Scheibe 22 weist dabei ebenfalls einen radialen Schlitz auf, in dem ein Stift 23 der Blendenplatte 20 verschiebbar geführt ist.

Zur Verstellung der Blendenplatten 10, 20 erfolgt eine Relativverdrehung der Scheiben 19, 22 gegenüber der Scheibe 11. Werden alle drei Scheiben 11, 19, 22 deckungsgleich und synchron zueinander gedreht, so wird die Lage des eingeblendeten Schlitzes ebenfalls gedreht. Die Scheiben 11, 19, 22 sind zu ihrer individuellen bzw. synchronen Drehung mit Außenverzahnungen versehen. Die Stifte 17, 23 sind an nach unten bzw. oben abgekröpften Ansätzen der Blendenplatten 10, 20 befestigt.

Wesentlich für die dargestellte Primärstrahlenblende ist, daß die Blendenplatten 10, 20 aneinander vorbeibewegt werden können, so daß sie das Röntgenstrahlenbündel wahlweise mit einer von zwei Kanten begrenzen. Diese beiden Kanten sind für die Blendenplatte 10 mit 14, 15 bezeichnet. Die Blendenplatte 20 ist spiegelbildlich zur Blendenplatte 10 ausgeführt, d.h., die geradlinig verlaufende Kante liegt dabei in der in der Figur 3 gezeichneten Stellung links und die der Herzkontur angepaßte Kante rechts.

## Patentansprüche

1. Primärstrahlenblende für Röntgenuntersuchungsgeräte mit mindestens einem Paar gegenläufig zueinander verstellbarer Blendenplatten (10, 20), die in zwei im Abstand voneinander liegenden Ebenen verstellbar gelagert sind und deren Stellweg so groß gewählt ist daß sie aneinander vorbeibewegt werden können wobei durch jede Blendenplatte (10, 20) das Röntgenstrahlenbündel (2) wahlweise mit einer von zwei quer zum Stellweg liegenden und unterschiedlichen Formen aufweisenden Kanten (14, 15) begrenzbar ist, dadurch gekennzeichnet, daß für jede der Blendenplatten (10, 20) individuelle Stellmittel (19, 22) vorgesehen sind.

## Claims

1. Primary radiation shutter for x-ray examination apparatus having at least one pair of shutter plates (10, 20) capable of being adjusted relative to each other, which plates are mounted so that they are adjustable in two planes spaced from each other and the adjustment distance of which is selected to be so sized that they are able to be moved past each other, wherein by means of each shutter plate (10, 20) the bundle of x-rays can be limited selectively, with one of two edges (14, 15) lying transverse to the adjustment distance and having different shapes, characterised in that individual adjusting means (19, 22) are provided for each of the shutter plates (10, 20).

## Revendications

1. Diaphragme pour le rayonnement primaire pour des appareils de radiodiagnostic, comportant au moins un couple de plaques (10, 20), qui sont déplaçables en sens inverse l'une par rapport à l'autre et sont montées de manière à être déplaçables dans deux plans distants l'un de l'autre et dont la voie de réglage est choisie suffisamment longue pour qu'elles puissent se déplacer l'une devant l'autre, le faisceau (2) de rayons X pouvant être limité au choix par l'un de deux bords (14, 15) de chaque plaque (10, 20) du diaphragme, qui sont disposés transversalement par rapport à la voie de réglage et possèdent des formes différentes, caractérisé par le fait que des moyens individuels de réglage (19, 22) sont prévus pour chacune des plaques (10, 20) du diaphragme.

FIG 1

FIG 2

FIG 3